# EUROPEAN PATENT SPECIFICATION

(11) **EP 1 669 459 B1**
(45) Date of publication and mention of the grant of the patent: **22.10.2014**
(21) Application number: 04788382.2
(22) Date of filing: 30.09.2004
(51) Int. Cl.: C12P 7/46, C12N 15/00

(54) **METHOD OF PURIFYING SUCCINIC ACID FROM FERMENTATION LIQUID**
VERFAHREN ZUR AUFREINIGUNG VON BERNSTEINSÄURE AUS FERMENTATIONSFLÜSSIGKEIT
PROCEDE DE PURIFICATION D'ACIDE SUCCINIQUE A PARTIR D'UN LIQUIDE DE FERMENTATION

(30) Priority: 30.09.2003 JP 2003340091
(43) Date of publication of application: 14.06.2006
(73) Proprietor: Ajinomoto Co., Inc., Tokyo 104-8315 (JP)
(72) Inventor: KUSHIKU, Takeshi, Ajinomoto Co., Inc., Kawasaki-shi, Kanagawa 210-8681 (JP); FUJIWARA, Kenji, Ajinomoto Co., Inc., Kawasaki-shi, Kanagawa 210-8681 (JP); SATOU, Takeru, Ajinomoto Co., Inc., Kawasaki-shi, Kanagawa 210-8681 (JP); SANO, Chiaki, Ajinomoto Co., Inc., Kawasaki-shi, Kanagawa 210-8681 (JP)
(74) Representative: Vossius & Partner
(86) International application number: PCT/JP2004/014354
(87) International publication number: WO 2005/030973

(56) References cited:
- EP-A1- 0 389 103
- EP-A1- 0 405 707
- EP-B1- 0 410 728
- JP-A- 3 151 884
- US-A- 5 143 834
- US-A- 5 168 055
- MAXA E. ET AL.: 'Modified HPLC method for the routine quantitation of major organic acids in wine, must and fruit juices' MITTEILUNGEN KLOSTERNEUBURG vol. 41, no. 6, 1991, pages 233 - 237, XP002984213
- CALVARY E.C.: 'Separation of vanillylmandelic (VMA) and homovanillic (HVA) acids from urine for assay by a two resin column system' MICROCHEMICAL JOURNAL vol. 23, no. 4, 1978, pages 473 - 480, XP002984214

## Description

### Technical Field

The present invention relates to a technique for purifying succinic acid from a fermentation broth in which a salt of succinic acid is accumulated.

### Background Art

In recent years, succinic acid draws attention as a raw material of biodegradable polymers. Further, succinic acid is widely used as a raw material of special chemical products as a 4-carbon intermediate. To use succinic acid as a raw material of biodegradable polymers and special chemical products, it is necessary to produce succinic acid of high purity at a low cost. This is because impurities contained in the raw material succinic acid may inhibit a reaction for producing a final product from succinic acid or degrade quality of the final product.

Accordingly, in order to produce succinic acid to be used as a raw material of polymers or special chemical products by fermentation or an enzymatic method, it is necessary to efficiently remove impurities from a fermentation broth or enzymatic reaction mixture containing a large amount of impurities and produce succinic acid at a low cost.

In the production of succinic acid by fermentation or an enzymatic method, a counter ion is generally added to a medium or an enzymatic reaction solution to maintain optimal pH. Therefore, succinic acid often exists in the form of a salt in a culture broth or an enzymatic reaction mixture in which succinic acid is accumulated. Therefore, the counter ion added to the fermentation medium or enzymatic reaction solution must be removed in order to produce succinic acid of high purity. Further, the fermentation broth contains a large amount of impurities such as other organic acids and amino acids, and these impurities also need to be efficiently removed.

As methods for producing succinic acid of high purity, methods using an ion-exchange resin, methods using a hardly soluble salt of succinic acid, methods utilizing electrodialysis and so forth have been reported so far.

The methods using an ion-exchange resin are roughly classified into methods using an anion-exchange resin and methods using a weakly acidic cation-exchange resin.

As the method using an anion-exchange resin, a method comprising bringing a raw material liquid containing a salt of succinic acid into contact with an anion-exchange resin to allow the resin to adsorb succinic acid and then eluting succinic acid with an organic solvent, aqueous ammonia or the like has been reported (refer to Patent documents 1 and 2). In this method, however, an organic solvent or alkali such as ammonia contained in the eluate must be removed and collected, and this makes the process complicated.

Further, a method comprising allowing a cation-exchange resin to adsorb a counter ion for succinic acid and collecting succinic acid as a through-flow solution has been reported (refer to Patent document 3). However, this method suffers from a problem that, since the ion-exchange resin to be used is a weakly acidic cation-exchange resin, the counter ion for succinic acid in the raw material liquid are limited to ammonia, and the method cannot be applied to salts of succinic acid with other counter ions.

As a method using a hardly soluble salt of succinic acid, a method comprising adding calcium ion to a raw material liquid containing a salt of succinic acid and collecting calcium succinate as a precipitate is known (Patent document 4). However, this method suffers from a problem that removal of calcium salt as by-product generated at the time of removal of calcium from the precipitate is complicated.

Patent documents 5 and 6 disclose methods utilizing electrodialysis. However, these methods suffer from a problem that, it is difficult to remove other organic acid (acetic acid) contained in the raw material liquid since it shows the same behavior as succinic acid.

Meanwhile, as a method for collecting an organic acid such as succinic acid from an aqueous solution containing the organic acid, a method of adjusting a concentration of hydrogen ion in the aqueous solution to a level required to bind an anion of organic acid or more by using an H-type ion-exchange resin has been reported (Patent document 7). It is described that, when the concentration of hydrogen ion is adjusted to the level required to bind an anion of organic acid in this method, a concentration higher than the equivalent concentration by 1 to about 10% is preferable. However, it is difficult to substantially completely remove cation contained in a fermentation broth as counter ion for succinic acid even if the concentration of hydrogen ion is adjusted to such a concentration, and therefore a problem arises that purity of succinic acid decreases.
[Patent document 1] JP 62-238231A
[Patent documerit 2] U.S. Patent No. 5,132,456
[Patent document 3] JP 62-238232A
[Patent document 4] JP 62-294090A (see also US 5,143,834, EP 0389103, EP 0405707, US 5,168,055)
[Patent document 5] JP 02-283289A (see also US 5,143,834, EP 0389103, EP 0405707, US 5,168,055)
[Patent document 6] JP 03-151884A (see also EP 0410728B1)
[Patent document 7] WO01/66508

### DISCLOSURE OF THE INVENTION

An object of the present invention is to provide a method for purifying succinic acid of high purity with good yield from a fermentation broth or an enzymatic reaction mixture in which a salt of succinic acid is accumulated.

The inventors of the present invention assiduously studied in order to solve the aforementioned object. As a result, they found that impurities in a succinic acid-containing liquid could be efficiently removed by combining ion-exchange using a certain amount or more of an H-type strongly acidic cation-exchange resin with crystallization of succinic acid, and thereby succinic acid could be produced with high purity and good yield.

That is, the present invention provides the followings.
(1) A method for purifying succinic acid from a succinic acid-containing liquid containing cation which is obtained by fermentation or an enzymatic method, which comprises adding an acid to the succinic acid-containing liquid to adjust to pH 5.0 or lower, then bringing the succinic acid-containing liquid into contact with an H-type strongly acidic cation-exchange resin in an amount equivalent to or more than the amount of cation other than hydrogen ion contained in the succinic acid-containing liquid, and precipitating a crystal of succinic acid from the obtained ion-exchange-treated liquid to obtain purified succinic acid, wherein the succinic acid-containing liquid contains a salt of carbonic acid.
(2) The method according to (1), wherein the succinic acid-containing liquid contains a salt of succinic acid.
(3) The method according to (2), wherein the salt of succinic acid is selected from sodium succinate, potassium succinate, magnesium succinate, calcium succinate and ammonium succinate.
(4) The method according to (1), wherein the ion-exchange-treated liquid is used as the acid.
(5) The method according to (1), wherein the ion-exchange-treated liquid from which the precipitated succinic acid has been removed is used as the acid.
(6) A method for producing succinic acid, which comprises allowing bacterial cells or treated bacterial cells to act on an organic raw material in an aqueous reaction solution to obtain a succinic acid-containing liquid, adding an acid to the succinic acid-containing liquid to adjust to pH 5.0 or lower, then bringing the succinic acid-containing liquid into contact with an H-type strongly acidic cation-exchange resin in an amount equivalent to or more than the amount of cation other than hydrogen ion contained in the succinic acid-containing liquid, and precipitating a crystal of succinic acid from the obtained ion-exchange-treated liquid to obtain purified succinic acid, wherein the succinic acid-containing liquid contains a salt of carbonic acid.
(7) The method according to (6), wherein the aqueous reaction solution is neutralized with magnesium carbonate or magnesium hydroxide.

### DESCRIPTION OF THE PREFERRED EMBODIMENTS

The method described herein is a method for purifying succinic acid from a succinic acid-containing liquid containing a cation which is obtained by fermentation or an enzymatic method, which comprises bringing the succinic acid-containing liquid into contact with an H-type strongly acidic cation-exchange resin in an amount equivalent to or more than the amount of cation other than hydrogen ion contained in the succinic acid-containing liquid, and precipitating a crystal of succinic acid from the obtained ion exchange-treated liquid to obtain purified succinic acid.

Hereafter, the present invention will be explained in detail.

### <1> Preparation of succinic acid-containing liquid

The succinic acid-containing liquid containing cation which is obtained by fermentation or an enzymatic method to which the present invention is applied contains succinic acid and cation other than hydrogen ion. Specific examples thereof include fermentation broth containing succinic acid, and a reaction mixture obtained by using a bacterium that catalyzes a reaction to produce succinic acid from a carbon source or a raw material or intermediate of succinic acid synthesis, treated bacterial cells, an enzyme and so forth.

The succinic acid-containing liquid may contain a salt of succinic acid or a neutral salt of succinic acid. Specific examples of the salt include sodium succinate, potassium succinate, magnesium succinate, calcium succinate, ammonium succinate and so forth.

As a method for producing the succinic acid-containing liquid in the present invention , the methods described in JP 05-68576A and JP11-196888A can be used. Specifically, a succinic acid-containing liquid can be obtained by allowing cells of a bacterium that belongs to the genus *Brevibacterium* and has a succinic acid-producing ability or treated bacterial cells to act on an aqueous reaction solution containing an organic raw material such as fumaric acid or a salt thereof.

Examples of the aforementioned microorganism include aerobic coryneform bacteria such as the *Brevibacterium flavum* MJ-233 strain, the *Brevibacterium flavum* MJ-233-AB-41 strain and so forth.

The MJ-233 strain was deposited at the National Institute of Bioscience and Human-Technology, Agency of Industrial Science and Technology, Ministry of International Trade and Industry (Central 6, 1-1, Higashi 1-Chome, Tsukuba-shi, Ibaraki-ken, 305-8566, Japan) on April 28, 1975, and given an accession number of FERM P-3068. Then, the deposition was converted to an international deposition under the provisions of the Budapest Treaty on May 1, 1981, and given an accession number of FERM BP-1497. The MJ-233-AB-41 strain was deposited at the National Institute of Bioscience and Human-Technology, Agency of Industrial Science and Technology, Ministry of International Trade and Industry (Central 6, 1-1, Higashi 1-Chome, Tsukuba-shi, Ibaraki-ken, 305-8566, Japan) on November 17, 1976, and given an accession number of FERM P-3812. Then, the deposition was converted to an international deposition under the provisions of the Budapest Treaty on May 1, 1981, and given an accession number of FERM BP-1498.

The microorganism to be used in the present invention may belong to the genus *Brevibacterium* so long as it produces succinic acid. Examples of such a microorganism include microorganisms belonging to the genus *Candida* (JP 56-17077B), or *Anaerobiospirillum succiniciproducens.*

The aforementioned reaction solution may be added with reduced and/or oxidized nicotinamide adenine dinucleotide. The concentration to be added is 0.1 to 50 mM, or may be 1 to 40 mM.

The microorganisms such as aerobic coryneform bacteria may be modified so that intracellular pyruvate carboxylase (PC) activity is enhanced. For example, the PC activity can be enhanced by transforming a microorganism with a gene coding for PC. Examples of the PC gene to be used include genes derived from microorganisms, animals or plants, and more specifically, genes derived from human, mouse, rat, yeast or microorganisms belonging to the genus *Corynebacterium, Bacillus, Rhizobium* or *Escherichia.* Acquisition of PC gene and succinic acid-producing bacteria which is introduced with the PC gene are described in JP 11-196888A. Further, it is also expected that the succinic acid-producing ability is improved by enhancing phosphoenol pyruvate carboxylase activity of a microorganism (JP 07-83714B, JP 09-121872A).

Specific examples of the PC gene and plasmid containing the gene include pPC-PYC2 and the PC gene (PYC2) contained in this plasmid, which is derived from *Saccharomyces cerevisiae,* as described in JP 11-196888A. Other PC gene (PYC1) derived from *Saccharomyces cerevisiae* can also be used.

When an aerobic coryneform bacterium, in particular, a bacterium introduced with the PC gene is used, and such a bacterium or treated cells thereof is allowed to act on an aqueous reaction solution containing an organic raw material, the reaction solution may contain carbonate ion, hydrogencarbonate ion or carbon dioxide gas. Further, this reaction may be performed under an anaerobic condition.

In order to use an aerobic coryneform bacterium for the method of the present invention, bacterial cells can be used after culturing under a general aerobic condition. As a medium to be used for culture, media which are used for conventional culture of microorganisms can be employed. For example, a common medium obtained by adding natural nutrients such as meat extract, yeast extract and peptone into a composition comprising inorganic salts such as ammonium sulfate, potassium phosphate and magnesium sulfate can be used.

After culture, bacterial cells are collected by centrifugation or membrane separation or the like, and used as they are or as treated bacterial cells, for the reaction described below. The term "treated bacterial cells" used herein means, for example, bacterial cells immobilized with acrylamide, carrageenan , disrupted cells, a centrifugation supernatant thereof, or a fraction having the PC activity obtained by partially purifying the supernatant by a treatment with e.g. ammonium sulfate.

For the reaction solution, water, buffer or medium is used, and the medium may contain suitable inorganic salts.

The organic raw material to be used for the present invention can be converted to succinic acid in fermentation, and can be selected from common organic raw materials. Specifically, glucose and ethanol, which are inexpensive and provide a high production rate of succinic acid, are used. In this case, the concentration of glucose to be added may be 0.5 to 500 g/L, and that of ethanol may be 0.5 to 30 g/L.

Further, examples of organic raw materials to be used in enzymatic methods include fumaric acid or a salt thereof such as sodium fumarate and ammonium fumarate.

When living microbial cells are used for the production of the succinic acid-containing liquid, it is difficult to strictly distinguish whether succinic acid is produced by fermentation or an enzymatic reaction. So long as a liquid containing a cation and succinic acid can be obtained as a result, it is not a problem in the present invention whether succinic acid is produced by fermentation or an enzymatic reaction.

Generally, cation as a counter ion for succinic acid may be added in order to maintain optimal pH of the reaction solution. However, such a counter ion may not be necessarily added. The counter ion for succinic acid does not inhibit fermentation or enzymatic reaction. As a counter ion for succinic acid, sodium ion, potassium ion, magnesium ion, calcium ion, ammonium ion or combination thereof is usually used, and sodium ion, potassium ion, magnesium ion, ammonium ion or combination thereof may be used.

The reaction solution may be neutralized with magnesium carbonate and/or magnesium hydroxide, because production rate and yield of succinic acid can be improved. The reaction solution may be maintained at pH 5 to 10, or at pH 6 to 9.5.

When a reaction solution containing carbonate ion or hydrogencarbonate ion or carbon dioxide gas is used for the preparation of the succinic acid-containing liquid, carbonate ion or hydrogencarbonate ion is added at a concentration of 1 to 500 mM, 2 to 300 mM, or 3 to 200 mM. When carbon dioxide gas is contained, 50 mg to 25 g, 100 mg to 15 g, or 150 mg to 10g, of carbon dioxide gas is contained per 1 L of the solution.

Further, the aforementioned anaerobic condition means that the reaction is performed while maintaining a low concentration of dissolved oxygen in the solution. The reaction may be performed at a dissolved oxygen concentration of 0 to 2 ppm, 0 to 1 ppm, or 0 to 0.5 ppm. Examples of methods for such a reaction include a reaction in a sealed vessel with no ventilation, a reaction with supply of an inert gas such as a nitrogen gas, a method using ventilation with an inert gas containing carbon dioxide gas, and so forth.

The reaction is usually performed at a temperature of 15 to 45°C, and may also be performed at 25 to 37°C. The reaction is performed in the pH range of 5 to 9, and may also be performed at pH 6 to 8. The reaction is usually performed for 5 to 120 hours. The amount of bacterial cells to be used for the reaction may be in an amount of I to 700 g/L, 10 to 500 g/L,or 20 to 400 g/L. When treated bacterial cells are used, they may be used in an amount corresponding to the aforementioned amount of bacterial cells.

Examples of the treated bacterial cells mentioned above include bacterial cells immobilized with acrylamide, carrageenan , disrupted bacterial cells, a centrifugation supernatant thereof, a fraction obtained by purifying the supernatant by a treatment with e.g. ammonium sulfate.

The succinic acid-containing liquid obtained as described above usually contains a cation as a counter ion for succinic acid, organic acids other than succinic acid, amino acids, inorganic salts, proteins, saccharides, lipids, bacterial cells, and so forth.

### <2> Ion-exchange

In the present invention, the succinic acid-containing liquid obtained as described above is brought into contact with an H-type strongly acidic cation-exchange resin in an amount equivalent to or more than the amount of cation other than hydrogen ion contained in the succinic acid-containing liquid, and crystal of succinic acid is precipitated from the obtained ion-exchange-treated liquid to obtain purified succinic acid.

Prior to the ion-exchange or crystallization process, bacterial cells or treated bacterial cells may be separated from a fermentation broth or an enzymatic reaction mixture. In general, this procedure may be performed prior to the ion-exchange, but it may be performed after the ion-exchange and prior to the crystallization process. The method for separating bacterial cells or treated bacterial cells from the succinic acid-containing liquid may be any methods commonly used for separation of bacterial cells such as filtration, centrifugation and combination thereof can be employed.

Further, if a succinic acid-containing liquid that contains carbonate ion, hydrogencarbonate ion or carbon dioxide gas is brought into contact with an H-type strongly acidic ion-exchange resin, pH of the liquid is lowered as a result of the exchange of hydrogen ion and cation, and carbonate ion and hydrogencarbonate ion receive hydrogen ion and are released as carbon dioxide. Because the liquid may strongly sparkle at this time, the procedure of ion-exchange may become very difficult. Therefore, an acid may be added to the succinic acid-containing liquid prior to the ion-exchange to make the liquid acidic and thereby cause decarbonylation.

The aforementioned acidic condition is preferably pH 5.0 or lower or may be at pH 4.8 or lower. The acid to be added may be an inexpensive acid such as hydrochloric acid and sulfuric acid. Further, as the acid to be added, the succinic acid-containing liquid after the ion-exchange operation as described later (ion-exchange-treated liquid), or the ion-exchange-treated liquid from which precipitated succinic acid has been removed (mother liquor after removal of crystallized succinic acid) can be used. Since these ion-exchange-treated liquid and mother liquor have a low pH, they are effective for lowering pH of the fermentation broth. Furthermore, they have an advantage that they neither decrease the yield nor increase the cost for disposal of waste acid, because the added liquid can be recycled by repeating the processes of the method of the present invention.

The succinic acid-containing liquid, the succinic acid-containing liquid subjected to a cell removal treatment and, if necessary, a decarbonylation treatment, is brought into contact with an H-type cation-exchange resin. By this ion-exchange procedure, cation and amino acids in the succinic acid-containing liquid are adsorbed on the ion-exchange resin and thereby removed. The inventors of the present invention found that not only basic amino acids but also neutral and acidic amino acids had been removed by this procedure. A fermentation broth usually has a pH around neutral. In this pH region, basic amino acids are positively charged, neutral amino acids are not dissociated, and acidic amino acids are negatively charged. Therefore, acidic and neutral amino acids are not adsorbed on the cation-exchange resin. However, if a raw material liquid containing salt of succinic acid is brought into contact with an H-type strongly acidic cation-exchange resin, the cation as counter ion for succinic acid are removed from the raw material liquid and replaced with hydrogen ion, resulting in decrease in pH. It is considered that, as a result, neutral and acidic amino acids are also positively charged and adsorbed on the cation-exchange resin.

The H-type strongly acidic cation-exchange resin to be used in the present invention may have either a low or high crosslinking level Further, those of either a gel type or porous type can be used. Specific examples thereof include commercially available DIAION SK 1B, SK104, SK110, PK212, PK216 (Mitsubishi Chemical Corporation), these products of other grades and so forth.

In the present invention, a strongly acidic ion-exchange resin must be converted to H-type before use. The method for conversion to H-type may be a commonly used method. Any of batch type method, single column type method and multiple column type method can be used (Kagaku Kogaku Binran (Chemical Engineering Handbook), Revised 4th Edition, Ed. by The Society of Chemical Engineers, Japan, Maruzen). Further, an acid is used for the conversion to H-type, and an acid having pKa lower than pK of the strongly acidic ion-exchange resin is selected. Further, it might be that the acid does not have oxidation ability, which causes degradation of the ion-exchange resin. Any acid can be used in the present invention so long as these requirements are satisfied. In general, hydrochloric acid or dilute sulfuric acid is used as the acid.

The method for bringing the succinic acid-containing liquid into contact with the H-type strongly acidic ion-exchange resin is not particularly limited, and usually used methods as described above can be employed. Any of batch type method, single column type method, and multiple column type method can be employed (Kagaku Kogaku Binran, Revised 4th Edition, Ed. by The Society of Chemical Engineers, Japan, Maruzen). A column method is usually employed, in which the succinic acid-containing liquid is passed through a column filled with the H-type strongly acidic ion-exchange resin, although a batch method can also be used.

In the present invention, the liquid obtained by bringing the succinic acid-containing liquid into contact with the H-type strongly acidic ion-exchange resin is defined as a through-flow liquid.

In the present invention, it is necessary that the exchange capacity of the H-type strongly acidic ion-exchange resin is equivalent to or more than the amount of cation other than hydrogen ion contained in the succinic acid-containing liquid. The term "cation other than hydrogen ion" used herein includes amino acids which are negatively charged during the ion-exchange treatment as described above as well as metal cation. Impurities in the succinic acid-containing liquid can be effectively removed by using an H-type strongly acidic ion-exchange resin having exchange capacity equivalent to or more than the amount of the cation. In particular, ions such as sodium ion, potassium ion, magnesium ion and ammonium ion, and amino acids such as serine, glutamic acid, alanine, valine, methionine and tyrosine, which are difficult to be completely removed by crystallization alone, can be efficiently removed. The total concentration of cation other than hydrogen ion in the through-flow liquid may be 1.0% or lower, or 0.5% or lower, with respect to succinic acid.

Among the cations other than hydrogen ion contained in the succinic acid-containing liquid or the through-flow liquid, the concentration of metal cation can be measured by, for example, an ion electrode method, atomic absorption method or ion chromatography and the concentration of amino acids can be measured by using e.g. an amino acid analyzer (Analytical Instrument Guide, 9th Edition, September 5, 2001, Japan Analytical Instruments Manufacturers Association).

### <3> Crystallization

The crystal of succinic acid is precipitated from the succinic acid-containing liquid which has been subjected to the ion-exchange. Examples of the method of precipitating the crystal include concentration, cooling of the ion-exchange-treated liquid, or addition of an organic solvent into the ion-exchange-treated liquid, or combination thereof. Purified succinic acid can be obtained by this procedure. In particular, organic acids other than succinic acid as well as carbonate ion, ammonium ion and so forth, which cannot be completely removed by crystallization, decarbonylation or ion-exchange, are efficiently removed by this procedure.

The condition of the concentration procedure may be vacuum concentration. However, concentration using reverse osmosis membrane (Kagaku Kogaku Binran, Revised 4th Edition, 1978, Maruzen), or concentration using electrodialysis (Food Membrane Technology, 1999, Korin) may also be employed.

The vacuum concentration may be performed under a pressure of 50 kPa or lower, 25 kPa or lower or particularly 15 kPa or lower.

To precipitate crystal of succinic acid by concentration procedure, the liquid is concentrated to a concentration higher than the solubility of succinic acid.

The solubility of succinic acid is described in, for example, Kagaku Binran Basic Part II, Revised 2nd Edition (The Chemical Society of Japan, 1975, Maruzen) and so forth.

To precipitate the crystal of succinic acid by cooling, the cooling is performed so that the liquid is cooled to a temperature at which the concentration of the succinic acid in the liquid becomes lower than the solubility of succinic acid.

The solubility of succinic acid is described in, for example, Kagaku Binran Basic Part II, Revised 2nd Edition (The Chemical Society of Japan, 1975, Maruzen) and so forth.

To precipitate the crystal of succinic acid by adding an organic solvent, an organic solvent such as methanol or ethanol is added.

Solubility of succinic acid in an organic solvent is described in, for example, Kagaku Binran Basic Part, II Revised 2nd Edition (The Chemical Society of Japan, 1975, Maruzen) and so forth.

Succinic acid can be crystallized by concentration, cooling or addition of an organic solvent alone, or combination of these.

Precipitated crystal is separated from the mother liquor in a conventional manner. Examples of the separation method include filtration, centrifugal filtration and centrifugal sedimentation.

### Examples

The present invention will be explained more specifically with reference to the following examples.

### Reference Example 1: Construction of PC gene-amplified strain

### <1> Cloning of a DNA fragment containing PC gene derived from yeast Saccharomyces cerevisiae (PYC2)

### (A) Extraction of a total DNA of Saccharomyces cerevisiae

The Saccharomyces cerevisiae W303-1A strain (Yeast, Vol. 2, pp.163-167 (1986)) was inoculated in 1 L of yeast growth medium (YPAD) [composition: 10 g of yeast extract, 20 g of peptone, 20 g of glucose, 100 mg of adenine and 1000 ml of distilled water] using a platinum loop and cultured until the late logarithmic growth phase at 30°C, and the cells were collected.

The obtained cells were suspended at a concentration of 10 mg/ml in 15 ml of a solution containing 10 mg/ml lysozyme, 10 mM NaCl, 20 mM Tris buffer (pH 8.0) and 1 mM EDTA·2Na (the concentration of each component is the final concentration). Then, the suspension was added with proteinase K at a final concentration of 100 µg/ml and incubated at 37°C for 1 hour. Then, the solution was added with sodium dodecylsulfate (SDS) at a final concentration of 0.5% and incubated at 50°C for 6 hours for lysis. This lysate was added with an equivalent amount of a phenol/chloroform solution and slowly shaken at room temperature for 10 minutes, followed by centrifugation (5,000 x g, 20 minutes, 10 to 12°C) to separate the supernatant fraction. This supernatant was added with sodium acetate at a concentration of 0.3 M and slowly added with 2-fold volume of ethanol. DNA that existed between the aqueous layer and the ethanol layer was taken up with a glass rod, washed with 70% ethanol, and air-dried. The obtained DNA was added with 5 ml of 10 mM Tris buffer (pH 7.5)/1 mM EDTA.2Na solution, left stand overnight at 4°C and used for the following experiments.

### (B) Cloning of a DNA fragment containing PC gene derived from Saccharomyces cerevisiae (PYC2) and construction of a recombinant strain

PCR was performed by using the chromosomal DNA prepared in (A) mentioned above as a template. For PCR, the following pair of primers were synthesized by using "394 DNA/RNA Synthesizer" manufactured by Applied Biosystems, and used.

(a-1) 5'-TTT CAT ATG AGC AGT AGC AAG AAA TTG-3' (SEQ ID NO: 1)
(b-1) 5'-TTT CCT GCA GGT TAA CGA GTA AAA ATT ACT TT-3' (SEQ ID NO: 2)

PCR was performed under the following condition by using "DNA Thermal Cycler" manufactured by Perkin Elmer Cetus and Recombinant TaqDNA Polymerase TaKaRa Taq (Takara Shuzo) as a reaction reagent.

### Reaction mixture:

| | |
|---|---|
| (10x) PCR buffer | 10 µl |
| 1.25 mM dNTP mixture | 16 µl |
| Template DNA | 10 µl |
| | (DNA content: 1 µM or lower) |
| a-1 and b-1 primers mentioned above | 1 µl each |
| | (final concentration: 0.25 µM) |
| Recombinant TaqDNA polymerase | 0.5 µl |
| Sterilized distilled water | 61.5 µl |

The above components were mixed, and 100 µl of the reaction mixture was used for PCR.

### PCR cycle:

Denaturation step: 94°C for 60 seconds
Annealing step: 52°C for 60 seconds
Extension step: 72°C for 120 seconds

The above cycle as one cycle was repeated 25 times.

10 µl of the reaction mixture obtained by the above reaction was subjected to electrophoresis using 0.8% agarose gel, and thereby a DNA fragment of about 3.56 kb could be detected.

### <2> Preparation of a recombinant coryneform bacterium using the PC gene

### (A) Construction of a shuttle vector

On the basis of the sequence of the region required for stabilization of a plasmid in a coryneform bacterium, which exists in the plasmid pCRY30 described in JP 03-210184A, the following pair of primers were synthesized by using "394 DNA/RNA Synthesizer" manufactured by Applied Biosystems.

(a-2) 5'-TTT CTC GAG CGC ATT ACC TCC TTG CTA CTG-3' (SEQ ID NO: 3)
(b-2) 5'-TTT GAA TTC GAT ATC AAG CTT GCA CAT CAA-3' (SEQ ID NO: 4)

The plasmid pCRY30 is a plasmid constructed as described below. That is, a DNA of plasmid pBY503 (refer to JP 01-95785A for details of this plasmid) is extracted from *Brevibacterium stationis* IFO12144, which was deposited at the National Institute of Bioscience and Human Technology, Agency of Industrial Science and Technology (currently, International Patent Organism Depositary, National Institute of Advanced Industrial Science and Technology (Tsukuba Central 6, 1-1, Higashi 1-chome, Tsukuba-shi, Ibaraki-ken 305-8566, Japan)) on July 18, 1988 as an accession number of FERM P-10136, and then converted to an international deposition under the provisions of the Budapest Treaty on July 18, 1988 and given an accession number of FERM BP-2515. Then, a DNA fragment having a size of about 4.0 kb and containing a gene responsible for replication (replication region) of the plasmid is excised with a restriction enzyme Xhol, and a DNA fragment having a size of about 2.1 kb and containing a gene responsible for stabilization (stabilization region) of the plasmid is excised with restriction enzymes EcoRI and KpnI. By incorporating both of these DNA fragments into each of the EcoRI-KpnI site and the Sall site of the plasmid pHSG298 (Takara Shuzo), the plasmid vector pCRY30 can be prepared.

PCR was performed under the following conditions by using "DNA Thermal Cycler" manufactured by Perkin Elmer Cetus and Recombinant TaqDNA Polymerase TaKaRa Taq (Takara Shuzo) as a reaction reagent.

### Reaction mixture:

| | |
|---|---|
| (10x) PCR buffer | 10 µl |
| 1.25 mM dNTP mixture | 16 µl |
| Template DNA | 10 µl |
| | (DNA content: 1 µM or lower) |
| a-2 and b-2 primers mentioned above | 1 µl each |
| | (final concentration: 0.25 µM) |
| Recombinant TaqDNA polymerase | 0.5 µl |
| Sterilized distilled water | 61.5 µl |

The above components were mixed, and 100 µl of this reaction mixture was used for PCR.

### PCR cycle:

Denaturation step: 94°C for 60 seconds
Annealing step: 52°C for 60 seconds
Extension step: 72°C for 120 seconds

The above cycle as one cycle was repeated 25 times.

10 µl of the reaction mixture obtained by the above reaction was subjected to electrophoresis using 0.8% agarose gel, and thereby a DNA fragment of about 1.1 kb could be detected.

10 µl of the reaction mixture in which an amplification product was confirmed above, and 1 µl of plasmid pBluescriptIISK+ were completely digested with restriction enzymes EcoRI and Xhol, respectively, and treated at 70°C for 10 minutes to inactivate the restriction enzymes, and then these were mixed, added with 1 µl of T4 DNA ligase 10x buffer and 1 unit of T4 DNA ligase, and sterilized distilled water to make 10 µl, and reacted at 15°C for 3 hours to ligate the fragments.

Using the obtained plasmid-containing solution, *Escherichia coli* JM109 (Takara Shuzo) was transformed by the calcium chloride method [Journal of Molecular Biology, 53, 159 (1970)] and spread over a medium [10 g of trypton, 5 g of yeast extract, 5 g of NaCl and 16 g of agar dissolved in 1 L of distilled water] containing 50 mg of ampicillin.

Strains which were capable of growing on this medium were subjected to liquid culture in a conventional manner, and the plasmid DNA was extracted from the culture solution, and the plasmid was digested with restriction enzymes (EcoRI, XhoI) to confirm an inserted fragment. As a result, an inserted DNA fragment having a length of 1.1 kb was identified in addition to the DNA fragment of the plasmid pBluescriptIISK+ having a length of 3.0 kb. This plasmid was named pBSpar.

On the basis of the sequence of the region required for replication of a plasmid in a coryneform bacterium, which exists in the plasmid pCRY31 described in U.S. Patent No. 5,185,262, the following 1 pair of primers were synthesized by using "394 DNA/RNA Synthesizer" manufactured by Applied Biosystems.

(a-3) 5'-TTT GGT ACC GAC TTA GAT AAA GGT CTA-3' (SEQ ID NO: 5)
(b-3) 5'-TTT CTC GAG TGC TGG TAAAAC AAC TTT-3' (SEQ ID NO: 6)

The aforementioned plasmid pCRY31 is a plasmid constructed as follows. That is, the plasmid pCRY3 (*Brevibacterium flavum* MJ233 GE102 harboring this plasmid was deposited at the National Institute of Bioscience and Human Technology, Agency of Industrial Science and Technology (currently, International Patent Organism Depositary, National Institute of Advanced Industrial Science and Technology (Tsukuba Central 6, 1-1, Higashi 1-chome, Tsukuba-shi, Ibaraki-ken 305-8566, Japan)) on January 8, 1988 as an accession number of FERM P-9802, and then converted to an international deposition under the provisions of the Budapest Treaty on January 8, 1988 and given an accession number of FERM BP-2513), which is obtained by ligating the aforementioned replication region derived from pBY503 into the plasmid pHSG398 (Takara Shuzo), is partially digested with Kpnl to obtain a DNA fragment. pBY503 is prepared from *Brevibacterium lactofermentum* IF012144 (FERM BP-2515) and completely digested with Kpnl to produce an about 7-kb DNA fragment. By ligating the above DNA fragments and selecting a plasmid that shows a digestion pattern mentioned in the following table when it is digested with the restriction enzymes, pCRY31 can be obtained.

**Table 1**

| Restriction enzyme | Number of recognition site | Length of fragments (kb) |
|---|---|---|
| KpnI | 3 | 7.0, 6.0, 2.2 |
| SauI | 2 | 10.0, 5.2 |
| PstI | 2 | 13.0, 2.2 |
| BamHI | 1 | 15.2 |

PCR was performed by using "DNA Thermal Cycler" manufactured by Perkin Elmer Cetus and Recombinant TaqDNA Polymerase TaKaRa Taq (Takara Shuzo) as a reaction reagent under the following condition.

### Reaction mixture:

| | |
|---|---|
| (10x) PCR buffer | 10 µl |
| 1.25 mM dNTP mixture | 16 µl |
| Template DNA | 10 µl |
| | (DNA content: 1 µM or lower) |
| a-3 and b-3 primers mentioned above | 1 µl each |
| | (final concentration: 0.25 µM) |
| Recombinant TaqDNA polymerase | 0.5 µl |
| Sterilized distilled water | 61.5 µl |

The above components were mixed, and 100 µl of this reaction mixture was used for PCR.

### PCR cycle:

Denaturation step: 94°C for 60 seconds
Annealing step: 52°C for 60 seconds
Extension step: 72°C for 120 seconds

The above cycle as one cycle was repeated 25 times.

10 µl of the reaction mixture obtained by the above reaction was subjected to electrophoresis using 0.8% agarose gel, and thereby a DNA fragment of about 1.8 kb could be detected.

10 µl of the reaction mixture in which an amplification product was confirmed above, and 1 µl of plasmid pBSpar were completely digested with restriction enzymes Xhol and Kpnl, and treated at 70°C for 10 minutes to inactivate the restriction enzymes, and then these were mixed, added with 1 µl of T4 DNA ligase 10x) buffer and 1 unit of T4 DNA ligase, and sterilized distilled water to make 10 µl, and reacted at 15°C for 3 hours to ligate the fragments.

Using the obtained plasmid-containing solution, *Escherichia coli* JM109 (Takara Shuzo) was transformed by the calcium chloride method [Journal of Molecular Biology, 53, 159 (1970)] and spread over a medium [10 g of trypton, 5 g of yeast extract, 5 g of NaCl and 16 g of agar dissolved in 1 L of distilled water] containing 50 mg of ampicillin.

Strains which were capable of growing on this medium were subjected to a liquid culture in a conventional manner, a plasmid DNA was extracted from the culture solution, and the plasmid was digested with restriction enzymes (XhoI, KpnI) to confirm an inserted fragment. As a result, an inserted DNA fragment having a length of 1.8 kb was identified in addition to the DNA fragment of plasmid pBSpar having a length of 4.1 kb. This plasmid was named pBSpar-rep.

1 µl of the plasmid pBSpar-rep prepared as described above and 1 µl of pHSG298 (Takara Shuzo) were completely digested with restriction enzymes KpnI and EcoRI, and treated at 70°C for 10 minutes to inactivate the restriction enzymes. Then, these were mixed, added with 1 µl of T4 DNA ligase 10x buffer and 1 unit of T4 DNA ligase, and sterilized distilled water to make 10 µl, and reacted at 15°C for 3 hours to ligate the fragments.

Using the obtained plasmid-containing solution, *Escherichia coli* JM109 (Takara Shuzo) was transformed by the calcium chloride method [Journal of Molecular Biology, 53, 159 (1970)] and spread over a medium [10 g of trypton, 5 g of yeast extract, 5 g of NaCl and 16 g of agar dissolved in 1 L of distilled water] containing 50 mg of kanamycin.

Strains which were capable of growing on this medium were subjected to liquid culture in a conventional manner, a plasmid DNA was extracted from the culture solution, and the plasmid was digested with restriction enzymes to confirm an inserted fragment. As a result, an inserted DNA fragment having a length of 2.9 kb was identified in addition to the DNA fragment of the plasmid pHSG298 having a length of 2.6 kb. This plasmid was named pHSG298par-rep.

### (B) Insertion of a tac promoter

In order to amplify a tac promoter fragment by PCR using a plasmid pTrc99A (Pharmacia) containing a tac promoter as a template, the following 1 pair of primers were synthesized by "394 DNA/RNA Synthesizer" manufactured by Applied Biosystems).

(a-4) 5'-TTT GGT ACC GAT AGC TTA CTC CCC ATC CCC-3' (SEQ ID NO: 7)
(b-4) 5'-TTT GGA TCC CAA CAT ATG AAC ACC TCC TTT TTA TCC GCT CAC AAT TCC ACA CAT-3' (SEQ ID NO: 8)

PCR was performed by using "DNA Thermal Cycler" manufactured by Perkin Elmer-Cetus and Recombinant TaqDNA Polymerase TaKaRa Taq (Takara Shuzo) as a reaction reagent under the following condition.

### Reaction mixture:

| | |
|---|---|
| (10x) PCR buffer | 10 µl |
| 1.25 mM dNTP mixture | 16 µl |
| Template DNA | 10 µl |
| | (DNA content: 1 µM or lower) |
| a-4 and b-4 primers mentioned above | 1 µl each |
| | (final concentration: 0.25 µM) |
| Recombinant TaqDNA polymerase | 0.5 µl |
| Sterilized distilled water | 61.5 µl |

The above components were mixed, and 100 µl of this reaction mixture was used for PCR.

### PCR cycle:

Denaturation step: 94°C for 60 seconds
Annealing step: 52°C for 60 seconds
Extension step: 72°C for 120 seconds

The above cycle as one cycle was repeated 25 times.

10 µl of the reaction mixture obtained by the above reaction was subjected to electrophoresis using 3% agarose gel, and thereby a DNA fragment of about 100 bp could be detected.

10 µl of the reaction mixture in which an amplification product was confirmed above and 5 µl of the plasmid pHSG298par-rep prepared in (A) mentioned above were completely digested with restriction enzymes BamHI and KpnI, and treated at 70°C for 10 minutes to inactivate the restriction enzymes. Then, these were mixed, added with 1 µl of T4 DNA ligase 10x) buffer and 1 unit of T4 DNA ligase, and sterilized distilled water to make 10 µl, and reacted at 15°C for 3 hours to ligate the fragments.

Using the obtained plasmid-containing solution, *Escherichia coli* JM109 (Takara Shuzo) was transformed by the calcium chloride method [Journal of Molecular Biology, 53, 159 (1970)] and spread over a medium [10 g of trypton, 5 g of yeast extract, 5 g of NaCl and 16 g of agar dissolved in 1 L of distilled water] containing 50 mg of kanamycin.

Strains which were capable of growing on this medium were subjected to liquid culture in a conventional manner, a plasmid DNA was extracted from the culture solution, and the plasmid was digested with restriction enzymes to confirm an inserted fragment. As a result, an inserted DNA fragment having a length of 0.1 kb was identified in addition to the DNA fragment of the plasmid prepared in (A) mentioned above having a length of 5.5 kb. This plasmid was named pHSG298tac.

### (C) Insertion of the PC gene into a shuttle vector

<1> 10 µl of the reaction mixture in which an amplification product was confirmed in (B) mentioned above and 5 µl of the plasmid pHSG298tac prepared in (B) mentioned above were digested with restriction enzymes BglII and SseI or BamHI and SseI, and treated at 70°C for 10 minutes to inactivate the restriction enzymes. Then, these were mixed, added with 1 µl of T4 DNA ligase 10x buffer and 1 unit of T4 DNA ligase, and sterilized distilled water to make 10 µl and reacted at 15°C for 3 hours to ligate the fragments.

Using the obtained plasmid-containing solution, *Escherichia coli* JM109 (Takara Shuzo) was transformed by the calcium chloride method [Journal of Molecular Biology, 53, 159 (1970)] and spread over a medium [10 g of trypton, 5 g of yeast extract, 5 g of NaCI and 16 g of agar dissolved in 1 L of distilled water] containing 50 mg of kanamycin.

Strains which were capable of growing on this medium were subjected to liquid culture in a conventional manner, a plasmid DNA was extracted from the culture solution, and the plasmid was digested with restriction enzymes (SseI, Ndel) to confirm an inserted fragment. As a result, an inserted DNA fragment having a length of 3.56 kb was identified in addition to the DNA fragment of the plasmid prepared in (B) mentioned above having a length of 5.6 kb.

This plasmid was named pPC-PYC2.

### (D) Transformation of Brevibacterium flavum MJ-233-AB-41 strain

The above plasmid was introduced into *Brevibacterium flavum* MJ-233-AB-41 (FERM BP-1498) according to the method described in U.S. Patent No. 5,185,262.

### Example 1

Succinic acid was purified from a fermentation broth in which succinic acid diammonium salt was accumulated.

### Preparation of H-type strongly acidic cation-exchange resin

H-type cation-exchange resin was prepared as follows.

1.6 L of Na-type strongly acidic cation-exchange resin SK1BL (Mitsubishi Chemical Corporation) was filled in a column. 5 L of 1 mol/L hydrochloric acid was passed through the column at a flow rate of 1.6 L/hr to convert the resin to H-type. Subsequently, 20 L of pure water was passed through the column to wash the resin, and the resin was used for the following experiments.

### Production of succinic acid by fermentation

Succinic acid was produced by fermentation according to the following method.

400 mL of a medium which contains 100 g of glucose, 0.5 g of magnesium sulfate heptahydrate, 0.65 g of orthophosphoric acid, 14.3 mL of soybean protein hydrolysis solution (total nitrogen content: 35 g/L), 1.0 g of ammonium sulfate, 20 mg of ferrous sulfate heptahydrate, 20 mg of manganese sulfate hydrate, 1 mg of D-biotin, 1 mg of thiamin hydrochloride and 0.05 mL of anti-foam (GD-113, NOF Corporation) per 1 L was prepared, adjusted to pH 6.5 with 1 N KOH, poured into a 1-L jar fermenter and sterilized by heating at 120°C for 20 minutes. After cooling the medium, the *Brevibacterium flavum* MJ-233-AB-41 strain transformed with the plasmid pPC-PYC2 was inoculated in the medium, and the medium was maintained at 30°C. The culture was performed for 20 hours with aeration of 300 ml per minute and stirring at 700 rpm, while pH was adjusted to 7.6 with ammonia gas. 100 ml of the obtained culture solution was used for the following succinic acid fermentation.

79 ml of a succharide solution which contains 520 g of glucose and 2.6 g of magnesium sulfate heptahydrate per 1 L was prepared and sterilized by heating at 120°C for 20 minutes. 221 ml of a medium which contains 1.21 g of orthophosphoric acid, 5.39 mL of soybean protein hydrolysis solution (total nitrogen content: 35 g/L), 1.86 g of ammonium sulfate, 37.14 mg of ferrous sulfate heptahydrate, 37.14 mg of manganese sulfate hydrate, 1.86 mg of D-biotin, 1.86 mg of thiamin hydrochloride and 0.09 mL of anti-foam (GD-113) per 1 L was prepared, adjusted to pH 6.5 with 1 N KOH and then sterilized by heating at 120°C for 20 minutes. The sterilized saccharide solution and the sterilized medium were put into a 1-L jar fermenter, cooled, then added with 100 mL of the aforementioned culture broth to make the total volume 400 mL, and then maintained at 30°C. Succinic acid fermentation was performed for 24 hours with aeration of 20 ml per minute and stirring at 400 rpm, while the medium was adjusted to pH 7.6 with ammonia gas.

The above-described succinic acid fermentation was performed 9 times to obtain 3.6 L of culture broth having a succinic acid concentration of 25 g/L.

### Purification of a crystal of succinic acid from fermentation broth in which salt of succinic acid was accumulated

The obtained culture broth was sterilized by heating at 120°C for 20 minutes and then centrifuged at 5000 x g for 20 minutes to obtain 3.5 L of supernatant.

The obtained supernatant was passed through the aforementioned H-type cation-exchange resin to obtain 2.2 L of a through-flow liquid. The obtained through-flow liquid was concentrated by using a rotary evaporator until the concentration of succinic acid became 19.2% to precipitate a crystal of succinic acid and obtain a succinic acid slurry. The obtained succinic acid slurry was cooled to 10°C to precipitate the crystal of succinic acid. The crystal and the mother liquor were separated. The obtained crystal of succinic acid was resuspended in a saturated aqueous solution of succinic acid having a volume of 15 times the weight of the crystal to wash the crystal, and then the crystal was separated.

### Comparative Example 1

The fermentation broth in which succinic acid was accumulated in the same manner as in Example 1 was sterilized (120°C, 20 minutes), and the cells were removed (5000 x g, 20 minutes). This fermentation broth was concentrated by using a rotary evaporator so that succinic acid concentration became 234 g/L. The obtained concentrated liquid was adjusted to pH 2.2 with addition of sulfuric acid and cooled to 10 °C to precipitate crystal of succinic acid. Then, the crystal was separated in the same manner as in Example 1, and washed by reslurrying with a saturated aqueous succinic acid solution to obtain a washed crystal.

The analytical values of the crystals of succinic acid obtained in Example 1 and Comparative Example 1 are shown in Table 2.

**Table 2**

| Analytical values of the crystals of succinic acid obtained in Example 1 and Comparative Example 1 (unit = % by weight) | | |
|---|---|---|
| | Example 1 | Comparative Example 1 |
| Succinic acid | 99.8 | 96.43 |
| Citric acid | n.d. | n.d. |
| Malic acid | n.d. | 0.02 |
| Lactic acid | n.d. | n.d. |
| Aspartic acid | n.d. | n.d. |
| Threonine | n.d. | n.d. |
| Serine | n.d. | 0.009 |
| Glutamic acid | n.d. | 0.012 |
| Glycin | n.d. | n.d. |
| Alanine | n.d. | 0.011 |
| Valine | n.d. | 0.043 |
| Methionine | n.d. | 0.016 |
| Isoleucine | n.d. | n.d. |
| Leucine | n.d. | n.d. |
| Tyrosine | n.d. | 0.010 |
| Phenylalanine | n.d. | n.d. |
| Lysine | n.d. | n.d. |
| Histidine | n.d. | n.d. |
| Arginine | n.d. | n.d. |
| Ammonia | 0.0005 | 0.044 |

| | | |
|---|---|---|
| n.d. denotes ≤ 0.0001. | | |

As shown in Table 2, other organic acids and amino acids were hardly detected in the washed crystal of succinic acid in Example 1, whereas many amounts of neutral and basic amino acids were detected in those of Comparative Example 1. This suggests that neutral and basic amino acids are easily taken up into a crystal obtained by crystallization of succinic acid.

The crystal of succinic acid obtained in Comparative Example 1 had very low contents of organic acids. This suggests that organic acids are removed relatively well from a crystal by crystallization of succinic acid.

It is considered that, in Example 1, neutral and basic amino acids were removed by adsorption to the H-type strongly acidic cation-exchange resin, and therefore they were not detected in the crystal of succinic acid at all. To confirm this, the liquid supplied to the H-type strongly acidic cation-exchange resin column and the through-flow liquid in Example 1 were analyzed. The analytical results are shown in Table 3.

**Table 3**

| Analytical results of the liquid supplied to H-type strongly acidic cation-exchange resin and the through-flow liquid in Example 1 (unit: g/g-succinic acid) | | |
|---|---|---|
| | Supplied liquid | Through-flow liquid |
| Citric acid | n.d. | n.d. |
| Malic acid | 0.0288 | 0.0290 |
| Lactic acid | 0.0153 | 0.0162 |
| Acetic acid | 0.5249 | 0.5698 |
| Aspartic acid | 0.0204 | n.d. |
| Threonine | n.d. | n.d. |
| Serine | n.d. | n.d. |
| Glutamic acid | 0.0419 | n.d. |
| Glycin | 0.1000 | n.d. |
| Alanine | n.d. | n.d. |
| Valine | 0.1189 | n.d. |
| Methionine | n.d. | n.d. |
| Isoleucine | n.d. | n.d. |
| Leucine | n.d. | n.d. |
| Tyrosine | n.d. | n.d. |
| Phenylalanine | n.d. | n.d. |
| Lysine | n.d. | n.d. |
| Histidine | n.d. | n.d. |
| Arginine | n.d. | n.d. |
| Ammonia | 0.2581 | 0.00135 |

| | | |
|---|---|---|
| n.d. denotes ≤ 0.0001. | | |

As shown in Table 3, it can be seen that most of the various kinds of amino acids contained in the supplied liquid were removed in the through-flow liquid obtained from the H-type strongly acidic cation-exchange resin column. This suggests that the amino acids had been removed by adsorption to H-type strongly acidic cation-exchange resin, and as a result, they were hardly detected in the crystal of succinic acid as the objective product.

### Example 2

Crystal of succinic acid was purified from a fermentation broth in which disodium succinate and sodium hydrogencarbonate were simultaneously accumulated.

### Preparation of an H-type strongly acidic cation-exchange resin

4.7 L of H-type strongly acidic cation-exchange resin was prepared in the same manner as in Example 1.

### Production of disodium succinate and sodium hydrogencarbonate by fermentation

Fermentation simultaneously accumulating sodium succinate and sodium carbonate was performed according to the method described in Example 1, provided that pH was adjusted by using 2.5 mol/L aqueous solution of sodium carbonate instead of ammonia. As a result, 3.1 L of culture broth having a succinic acid concentration of 70 g/L was obtained.

### Purification of a crystal of succinic acid from fermentation broth

3.1 L of the obtained fermentation broth was subjected to cross flow filtration using MF membrane module (PELLICON-2 membrane module manufactured by Millipore, effective membrane area: 0.1 m2, pore size: 0.22 µm) for removing cells. When 2.6 L of filtrate was obtained, 1 L of pure water was added to the liquid circulating the membrane for dilution filtration. As a result, 3.6 L of filtrate from which cells were removed was obtained.

### Decarbonylation by addition of acid

The obtained liquid from which cells were removed was adjusted to pH 4.0 by addition of the through-flow liquid from the ion-exchange resin column and the mother liquor of the crystallization obtained in Example 1 to perform decarbonylation, and thereby a decarbonylated liquid was obtained.

### Loading of the liquid on H-type strongly acidic cation-exchange resin

The obtained decarbonylated liquid was passed through a column filled with the H-type strongly acidic cation-exchange resin at a flow rate of 4.7 L/hr to obtain 3.5 L of a through-flow liquid. After completion of the loading, 3.8 L of pure water was further passed through the column, and thereby 7.3 L in total of through-flow liquid was obtained.

### Crystallization of succinic acid

The obtained through-flow liquid in a volume of 7.3 L was passed through a granular active carbon column for decolorization, then the liquid was concentrated in the same manner as in Example 1 to a succinic acid concentration of 30% by weight, and the obtained slurry was stirred overnight at 10°C. Then, the crystal of succinic acid was separated in the same manner as in Example 1 and washed with a saturated aqueous solution of succinic acid to obtain a washed crystal of succinic acid.

The analytical values of the crystal of succinic acid obtained in Example 2 are shown below.

**Table 4. Analytical values of the crystal of succinic acid obtained in Example 2 (unit: wt%)**

| | Example 2 |
|---|---|
| Succinic acid | 99.9 |
| Citric acid | n.d. |
| Malic acid | n.d. |
| Lactic acid | n.d. |
| Aspartic acid | n.d. |
| Threonine | n.d. |
| Serine | n.d. |
| Glutamic acid | 0.002 |
| Glycin | n.d. |
| Alanine | n.d. |
| Valine | n.d. |
| Methionine | n.d. |
| Isoleucine | n.d. |
| Leucine | n.d. |
| Tyrosine | n.d. |
| Phenylalanine | n.d. |
| Lysine | n.d. |
| Histidine | n.d. |
| Arginine | n.d. |
| Ammonia | n.d. |

| | |
|---|---|
| n.d. denotes≤0.0001. | |

As shown in Table 4, even when a fermentation broth in which sodium succinate was accumulated was used as a raw material, succinic acid of extremely high purity could be obtained. Thus, the present invention can also be used for a fermentation broth containing a monovalent cation as counter ion for succinic acid.

### Comparative Example 2

In the same manner as in Example 2, crystal of succinic acid was purified by using a fermentation broth in which disodium succinate and sodium hydrogencarbonate were simultaneously accumulated as a raw material. In this example, the liquid was passed through an H-type strongly acidic ion-exchange resin without performing decarbonylation.

### Loading of the liquid on the H-type strongly acidic cation-exchange resin

The cells were removed from the aforementioned fermentation broth, and the obtained liquid from which cells had been removed was passed through the H-type cation-exchange resin column. As a result, the liquid sparkled at the upper surface of the cation-exchange resin column, and the ion-exchange operation could not be performed. Therefore, the crystallization process could not be performed.

### Example 3

### Production of succinic acid from fermentation broth containing magnesium succinate

Succinic acid was purified from a fermentation broth containing magnesium succinate as a divalent metal salt.

### Preparation of H-type strongly acidic cation-exchange resin and production of succinic acid by fermentation

In the same manner as in Example 1, 4.7 L of H-type strongly acidic cation-exchange resin was prepared.

Magnesium succinate fermentation was performed by the same culture method as in Example 1, provided that magnesium hydroxide was used as a neutralizer instead of ammonia. Specifically, 2.5 mol/L magnesium hydroxide slurry was used instead of ammonia for pH adjustment. The medium was maintained at pH 7.5 to 7.7. As a result, 1 L of culture broth having a succinic acid concentration of 50 g/L was obtained.

The obtained culture broth was sterilized by heating at 120°C for 20 minutes and centrifuged at 5000 x g for 15 minutes to precipitate bacterial cells and thereby obtain a supernatant. The obtained supernatant was passed through the H-type strongly acidic cation-exchange resin in the same manner as in Example 1, and the obtained through-flow liquid was vacuum concentrated to obtain a crystal of succinic acid.

The analytical values of the crystal of succinic acid obtained in Example 3 are shown in Table 5.

**Table 5. Analytical values of the crystal of succinic acid obtained in Example 3 (unit: % by weight)**

| | Example 3 |
|---|---|
| Succinic acid | 99.9 |
| Citric acid | n.d. |
| Malic acid | n.d. |
| Lactic acid | n.d. |
| Aspartic acid | n.d. |
| Threonine | n.d. |
| Serine | n.d. |
| Glutamic acid | n.d. |
| Glycin | n.d. |
| Alanine | n.d. |
| Valine | n.d. |
| Methionine | n.d. |
| Isoleucine | n.d. |
| Leucine | n.d. |
| Tyrosine | n.d. |
| Phenylalanine | n.d. |
| Lysine | n.d. |
| Histidine | n.d. |
| Arginine | n.d. |
| Ammonia | 0.0067 |

| | |
|---|---|
| n.d. denotes ≤ 0.0001. | |

As shown in Table 5, it was found that succinic acid of high purity could also be obtained from magnesium salt of succinic acid, which is a salt of divalent cation, according to the present invention. Thus, the present invention can be applied not only to a succinic acid-containing fermentation broth containing a monovalent cation as a counter ion, but also to a succinic acid-containing fermentation broth containing a divalent cation as a counter ion.

### Industrial Applicability

According to the present invention, succinic acid of high purity can be easily obtained from a succinic acid-containing liquid obtained by fermentation or an enzymatic method.

## Claims

1. A method for purifying succinic acid from a succinic acid-containing liquid containing cation which is obtained by fermentation or an enzymatic method, which comprises adding an acid to the succinic acid-containing liquid to adjust to pH 5.0 or lower, then bringing the succinic acid-containing liquid into contact with an H-type strongly acidic cation-exchange resin in an amount equivalent to or more than the amount of cation other than hydrogen ion contained in the succinic acid-containing liquid, and precipitating a crystal of succinic acid from the obtained ion-exchange-treated liquid to obtain purified succinic acid, wherein the succinic acid-containing liquid contains a salt of carbonic acid.

2. The method according to claim 1, wherein the succinic acid-containing liquid contains a salt of succinic acid.

3. The method according to claim 2, wherein the salt of succinic acid is selected from sodium succinate, potassium succinate, magnesium succinate, calcium succinate and ammonium succinate.

4. The method according to claim 1, wherein the ion-exchange-treated liquid is used as the acid.

5. The method according to claim 1, wherein the ion-exchange-treated liquid from which the precipitated succinic acid has been removed is used as the acid.

6. A method for producing succinic acid, which comprises allowing bacterial cells or treated bacterial cells to act on an organic raw material in an aqueous reaction solution to obtain a succinic acid-containing liquid, adding an acid to the succinic acid-containing liquid to adjust to pH 5.0 or lower, then bringing the succinic acid-containing liquid into contact with an H-type strongly acidic cation-exchange resin in an amount equivalent to or more than the amount of cation other than hydrogen ion contained in the succinic acid-containing liquid, and precipitating a crystal of succinic acid from the obtained ion-exchange-treated liquid to obtain purified succinic acid, wherein the succinic acid-containing liquid contains a salt of carbonic acid.

7. The method according to claim 6, wherein the aqueous reaction solution is neutralized with magnesium carbonate or magnesium hydroxide.

## Patentansprüche

1. Verfahren zum Aufreinigen von Bernsteinsäure aus einer Bernsteinsäure enthaltenden Flüssigkeit, die Kationen enthält, die durch Fermentation oder ein enzymatisches Verfahren erhalten wird, umfassend das Hinzufügen einer Säure zu der Flüssigkeit, die Bernsteinsäure enthält, um diese auf pH 5,0 oder geringer einzustellen, dann das Inkontaktbringen der Flüssigkeit, die Bernsteinsäure enthält, mit einem H-Typ, stark saurem Kationenaustauscher-Säulenmaterial in einer Menge, die gleich oder größer ist als die Menge an Kationen außer Wasserstoff-Ionen, die in der Flüssigkeit, die Bernsteinsäure enthält, enthalten ist, und das Präzipitieren eines Bernsteinsäure-Kristalls aus der erhaltenen, mit Ionenaustauscher behandelten Flüssigkeit, um gereinigte Bernsteinsäure zu erhalten, wobei die Flüssigkeit, die Bernsteinsäure enthält, ein Salz von Kohlensäure enthält.

2. Verfahren nach Anspruch 1, wobei die Flüssigkeit, die Bernsteinsäure enthält, ein Salz von Bernsteinsäure enthält.

3. Verfahren nach Anspruch 2, wobei das Salz der Bernsteinsäure ausgewählt ist aus Natriumsuccinat, Kaliumsuccinat, Magnesiumsuccinat, Calciumsuccinat und Ammoniumsuccinat.

4. Verfahren nach Anspruch 1, wobei die mit Ionenaustauscher behandelte Flüssigkeit als die Säure verwendet wird.

5. Verfahren nach Anspruch 1, wobei die mit Ionenaustauscher behandelte Flüssigkeit, aus der die präzipitierte Bernsteinsäure entfernt wurde, als die Säure verwendet wird.

6. Verfahren zum Herstellen von Bernsteinsäure, umfassend das Ermöglichen von Bakterienzellen oder behandelten Bakterienzellen auf ein organisches Rohmaterial in einer wässrigen Reaktionslösung einzuwirken, um eine Flüssigkeit, die Bernsteinsäure enthält, zu erhalten, das Hinzufügen einer Säure zu der Flüssigkeit, die Bernsteinsäure enthält, um diese auf pH 5,0 oder geringer einzustellen, dann das Inkontaktbringen der Flüssigkeit, die Bernsteinsäure enthält, mit einem H-Typ, stark saurem Kationenaustauscher-Säulenmaterial in einer Menge, die gleich oder größer ist als die Menge an Kationen außer Wasserstoff-Ionen, die in der Flüssigkeit, die Bernsteinsäure enthält, enthalten ist, und das Präzipitieren eines Bernsteinsäure-Kristalls aus der erhaltenen, mit Ionenaustauscher behandelten Flüssigkeit, um gereinigte Bernsteinsäure zu erhalten, wobei die Flüssigkeit, die Bernsteinsäure enthält, ein Salz von Kohlensäure enthält.

7. Verfahren nach Anspruch 6, wobei die wässrige Reaktionslösung mit Magnesiumcarbonat oder Magnesiumhydroxid neutralisiert wird.

## Revendications

1. Procédé de purification d'acide succinique à partir d'un liquide contenant de l'acide succinique et contenant un cation qui est obtenu par fermentation ou par un procédé enzymatique, qui comprend l'ajout d'un acide au liquide contenant de l'acide succinique pour l'ajuster à pH 5,0 ou moins, puis la mise en contact du liquide contenant de l'acide succinique avec une résine échangeuse de cations fortement acide de type H en une quantité équivalente ou supérieure à la quantité de cations autres que l'ion hydrogène contenue dans le liquide contenant de l'acide succinique, et la précipitation d'un cristal d'acide succinique à partir du liquide obtenu par traitement par échange ionique pour obtenir de l'acide succinique purifié, dans lequel le liquide contenant de l'acide succinique contient un sel d'acide carbonique.

2. Procédé selon la revendication 1, dans lequel le liquide contenant l'acide succinique contient un sel d'acide succinique.

3. Procédé selon la revendication 2, dans lequel le sel d'acide succinique est choisi parmi le succinate de sodium, le succinate de potassium, le succinate de magnésium, le succinate de calcium et le succinate d'ammonium.

4. Procédé selon la revendication 1, dans lequel le liquide traité par échange ionique est utilisé comme acide.

5. Procédé selon la revendication 1, dans lequel le liquide traité par échange ionique duquel a été retiré l'acide succinique précipité est utilisé comme acide.

6. Procédé de production d'acide succinique, qui comprend le fait de laisser des cellules bactériennes ou des cellules bactériennes traitées agir sur une matière première organique dans une solution de réaction aqueuse pour obtenir un liquide contenant de l'acide succinique, l'ajout d'un acide au liquide contenant de l'acide succinique pour l'ajuster à pH 5,0 ou moins, puis la mise en contact du liquide contenant de l'acide succinique avec une résine échangeuse de cations fortement acide de type H en une quantité équivalente ou supérieure à la quantité de cations autres que l'ion hydrogène contenue dans le liquide contenant de l'acide succinique, et la précipitation d'un cristal d'acide succinique à partir du liquide obtenu par traitement par échange ionique pour obtenir de l'acide succinique purifié, dans laquelle le liquide contenant de l'acide succinique contient un sel d'acide carbonique.

7. Procédé selon la revendication 6, dans lequel la solution de réaction aqueuse est neutralisée avec du carbonate de magnésium ou de l'hydroxyde de magnésium.


### SEQUENCE LISTING

<110> Ajinomoto Co.. Inc. Mitsubishi Chemical Corporation
<120> Method for Purifying Succinic Acid from Fermentation Broth
<130> C2880PC4140
<150> JP2003-340091
   <151> 2003-09-30
<160> 8
<170> Patent In Ver. 2.0
<210> 1
   <211> 27
   <212> DNA
   <213> Artificial Sequence
<220>
   <223> Description of Artificial Sequence: primer
<400> 1
   tttcatatga gcagtagcaa gaaattg 27
<210> 2
   <211> 32
   <212> DNA
   <213> Artificial Sequence
<220>
   <223> Description of Artificial Sequence: primer
<400> 2
   tttcctgcag gttaacgagt aaaaattact tt 32
<210> 3
   <211> 30
   <212> DNA
   <213> Artificial Sequence
<220>
   <223> Description of Artificial Sequence: primer
<400> 3
   tttctcgagc gcattacctc cttgctactg 30
<210> 4
   <211> 30
   <212> DNA
   <213> Artificial Sequence
<220>
   <223> Description of Artificial Sequence: primer
<400> 4
   tttgaattcg atatcaagct tgcacatcaa 30
<210> 5
   <211> 27
   <212> DNA
   <213> Artificial Sequence
<220>
   <223> Description of Artificial Sequence: primer
<400> 5
   tttggtaccg acttagataa aggtcta 27
<210> 6
   <211> 27
   <212> DNA
   <213> Artificial Sequence
<220>
   <223> Description of Artificial Sequence: primer
<400> 6
   tttctcgagt gctggtaaaa caacttt 27
<210> 7
   <211> 30
   <212> DNA
   <213> Artificial Sequence
<220>
   <223> Description of Artificial Sequence: primer
<400> 7
   tttggtaccg atagcttact ccccatcccc 30
<210> 8
   <211> 54
   <212> DNA
   <213> Artificial Sequence
<220>
   <223> Description of Artificial Sequence: primer
<400> 8
   tttggatccc aacatatgaa cacctccttt ttatccgctc acaattccac acat 54